(19) Europäisches Patentamt
European Patent Office
Office européen des brevets

(11) EP 3 375 783 A1

(12) **EUROPEAN PATENT APPLICATION**
published in accordance with Art. 153(4) EPC

(43) Date of publication:
**19.09.2018 Bulletin 2018/38**

(21) Application number: **16864095.1**

(22) Date of filing: **02.11.2016**

(51) Int Cl.:
***C07D 471/04*** *(2006.01)* ***A01N 43/90*** *(2006.01)*
***A01P 7/04*** *(2006.01)*

(86) International application number:
**PCT/JP2016/082575**

(87) International publication number:
**WO 2017/082132 (18.05.2017 Gazette 2017/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **11.11.2015 JP 2015221092**

(71) Applicant: **Sumitomo Chemical Company, Limited**
**Tokyo 104-8260 (JP)**

(72) Inventors:
- **OOHIRA, Daisuke**
  **Hyogo 665-8555 (JP)**
- **SUNAMURA Eiriki**
  **Tokyo 104-8260 (JP)**

(74) Representative: **Vossius & Partner**
**Patentanwälte Rechtsanwälte mbB**
**Siebertstrasse 3**
**81675 München (DE)**

(54) **CONDENSED HETEROCYCLIC COMPOUND**

(57) A condensed heterocyclic compound represented by formula (I) [wherein $R^1$ represents a hydrogen atom, a halogen atom, a C1-C3 alkyl group which may have optionally one or more halogen atoms, a C1-C3 alkoxy group, a C2-C4 alkoxycarbonyl group, $S(O)_mR^2$, $NR^3R^4$, a nitro group or a cyano group; $R^2$ represents a C1-C3 alkyl group; $R^3$ and $R^4$ independently represent a hydrogen atom or a C1-C3 alkyl group; and m and n independently represent 0, 1 or 2] or an N oxide thereof has an excellent noxious organism control effect.

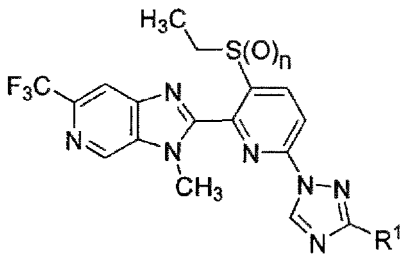

(I)

EP 3 375 783 A1

**Description**

TECHNICAL FIELD

The present invention is related to a certain class of fused heterocyclic compound and its use for controlling pests.

BACKGROUND ART

To date, some compounds for controlling pests have been developed and come into practical use. Also, a certain class of fused heterocyclic compound has been known (see Patent Document 1).

CITATION LIST

PATENT DOCUMENT

Patent Document 1: WO 2015/121136 pamphlet

SUMMARY OF THE INVENTION

(PROBLEMS TO BE SOLVED BY INVENTION)

An object of the present invention is to provide a compound having an excellent efficacy for controlling pests, and use of the compound for controlling pests.

(MEANS TO SOLVE PROBLEMS)

The present inventors have intensively studied to solve the above-mentioned problems, and as a result, find out that a fused heterocyclic compound represented by the below-mentioned formula (I) has an excellent control efficacy against pests.

The present invention provides the following embodiments.

[1] A fused heterocyclic compound represented by formula (I) or its N oxide compound:

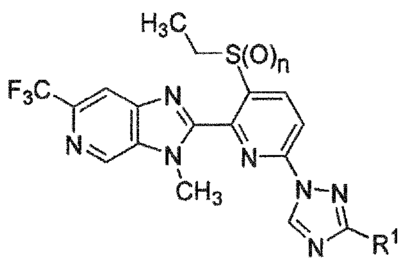

(I)

[wherein,

$R^1$ represents a hydrogen atom, a C1-C3 alkyl group which may have optionally one or more halogen atoms, a halogen atom, a C1-C3 alkoxy group, a C2-C4 alkoxycarbonyl group, $S(O)_mR^2$, $NR^3R^4$, a nitro group, or a cyano group,

$R^2$ represents a C1-C3 alkyl group,

$R^3$ and $R^4$ represent independently of each other a hydrogen atom or a C1-C3 alkyl group, and

m and n are independently of each other 0, 1 or 2.] (hereinafter, a fused heterocyclic compound represented by formula (I) or its N oxide compound is referred to as "Present compound" or "Compound of the present invention").

[2] A composition for controlling a pest comprising the compound according to [1] and an inert carrier.

[3] A method for controlling a pest, said method comprising applying an effective amount of the compound according to [1] to a pest or a habitat where a pest lives.

[EFFECT OF INVENTION]

The present compound has an excellent control efficacy against pests, and is thus useful as an active ingredient for an agent for controlling pests.

MODE FOR CARRYING OUT THE INVENTION

The substituent(s) as described herein is/are explained.

The term of "(which) may have optionally one or more halogen atoms" represents that when two or more halogen atoms are present, these halogen atoms may be identical to or different from each other.

The expression of "CX-CY" as used herein represents that the number of carbon atom is from X to Y. For example, the expression of "C1-C3" represents that the number of carbon atom is from 1 to 3.

The term of "halogen atom" represents fluorine atom, chlorine atom, bromine atom, or iodine atom.

Examples of "C1-C3 alkyl group that may have optionally one or more halogen atoms" include methyl, ethyl, propyl, isopropyl, fluoromethyl, difluoromethyl, trifluoromethyl, chlorodifluoromethyl, bromodifluoromethyl, 2,2,2-trifluoroethyl, perfluoroethyl, perfluoropropyl, and perfluoroisopropyl.

The term of "N oxide compound" represents a compound represented by the following formula (I-N):

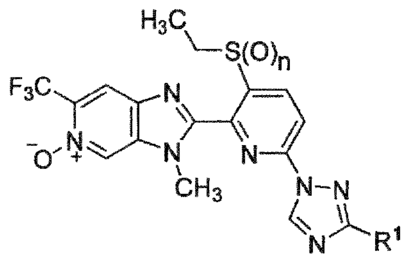

( I -N)

[wherein the symbols are the same as defined above.].

Embodiments of the compound of the present invention include the following compounds.

A compound of the present invention wherein $R^1$ represents a hydrogen atom, a halogen atom, a C1-C3 alkyl group which may have optionally one or more halogen atoms, a C1-C3 alkoxy group, or $NR^3R^4$;

A compound of the present invention wherein $R^1$ represents a hydrogen atom, a halogen atom, a C1-C3 alkyl group, or $NR^3R^4$;

A compound of the present invention wherein $R^1$ represents a hydrogen atom or $NR^3R^4$;

A compound of the present invention wherein $R^1$ represents a hydrogen atom or an amino group;

A compound of the present invention wherein $R^1$ represents a hydrogen atom or $NR^3R^4$, and n is 2.

Next, a process for preparing the compound of the present invention is explained.

The compound of the present invention can be prepared, for example, according to the following processes.

Process 1

A compound represented by formula (Ib) (hereinafter, referred to as Present compound (Ib)) and a compound represented by formula (Ic) (hereinafter, referred to as Present compound (Ic)) may be prepared by reacting a compound represented by formula (Ia) (hereinafter, referred to as Present compound (Ia)) with an oxidizing agent.

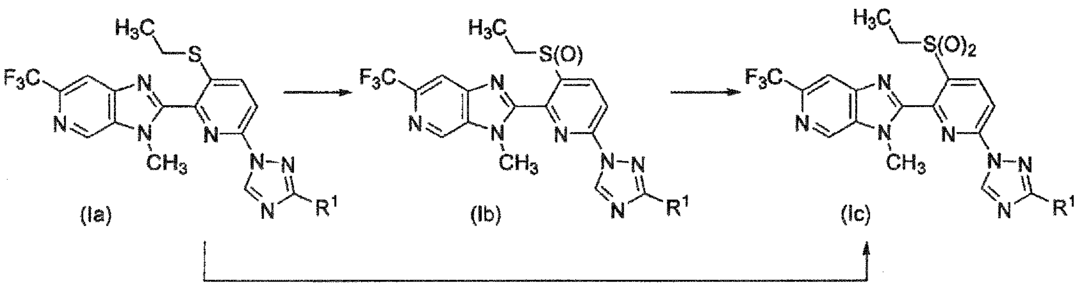

[wherein, the symbols are the same as defined above.]

First, a process for preparing the present compound (Ib) from the present compound (Ia) is described.

The reaction is usually carried out in a solvent. Examples of the solvent to be used in the reaction include halogenated hydrocarbons such as dichloromethane and chloroform (hereinafter, referred to as "halogenated hydrocarbons"), nitriles such as acetonitrile (hereinafter, referred to as "nitriles"), esters such as ethyl acetate, alcohols such as methanol and ethanol (hereinafter, referred to as "alcohols"), acetic acid, water, and mixtures thereof.

Examples of the oxidizing agent to be used in the reaction include sodium periodate, m-chloroperoxybenzoic acid (hereinafter, referred to as "mCPBA"), and hydrogen peroxide.

When hydrogen peroxide is used as the oxidizing agent, sodium carbonate or a catalyst may be added as needed.

Examples of the catalyst to be used in the reaction include tungstic acid, and sodium tungstate.

In the reaction, the oxidizing agent is used usually within a range of 1 to 1.2 molar ratio(s), sodium carbonate is used usually within a range of 0.01 to 1 molar ratio(s), and the catalyst is used usually within a range of 0.01 to 0.5 molar ratios, relative to 1 mole of the compound (Ia).

The reaction temperature is usually within a range of -20 to 80°C. The reaction period is usually within a range of 0.1 to 12 hours.

When the reaction is completed, water is added to reaction mixtures, and the reaction mixtures are extracted with organic solvent(s), and as needed, the organic layers are washed with an aqueous solution of a reducing agent (such as sodium sulfite, and sodium thiosulfate) and an aqueous solution of a base (such as sodium hydrogen carbonate) successively. The resulting organic layers are dried and/or concentrated to give the present compound (Ib).

Next, a process for preparing the present compound (Ic) from the present compound (Ib) is described.

The reaction is usually carried out in a solvent. Examples of the solvent to be used in the reaction include halogenated hydrocarbons, nitriles, alcohols, acetic acid, water, and mixtures thereof.

Examples of the oxidizing agent to be used in the reaction include mCPBA and hydrogen peroxide.

When hydrogen peroxide is used as oxidizing agent, sodium carbonate or a catalyst may be added as needed.

Examples of the catalyst to be used in the reaction include sodium tungstate.

In the reaction, the oxidizing agent is used usually within a range of 1 to 2 molar ratio(s), sodium carbonate is used usually within a range of 0.01 to 1 molar ratio(s), and the catalyst is used usually within a range of 0.01 to 0.5 molar ratios, relative to 1 mole of the present compound (Ib).

The reaction temperature is usually within a range of -20 to 120°C. The reaction period is usually within a range of 0.1 to 12 hours.

When the reaction is completed, water is added to reaction mixtures, and the reaction mixtures are extracted with organic solvent(s), and as needed, the organic layers are washed with an aqueous solution of a reducing agent (such as sodium sulfite, and sodium thiosulfate) and an aqueous solution of a base (such as sodium hydrogen carbonate) successively. The resulting organic layers are dried and/or concentrated to give the present compound (Ic).

Also, the present compound (Ic) may be prepared in one step (one-spot) by reacting the present compound (Ia) with an oxidizing agent.

The reaction may be carried out by using the oxidizing agent usually in 2.0 to 2.4 molar ratios relative to 1 mole of the present compound (Ia) according to a method for preparing the present compound (Ic) from the present compound (Ib).

Process 2

The compound of the present invention may be prepared by reacting a compound represented by formula (M1) (hereinafter, referred to as "compound (M1)") with a compound represented by formula (M2) (hereinafter, referred to as "compound (M2)").

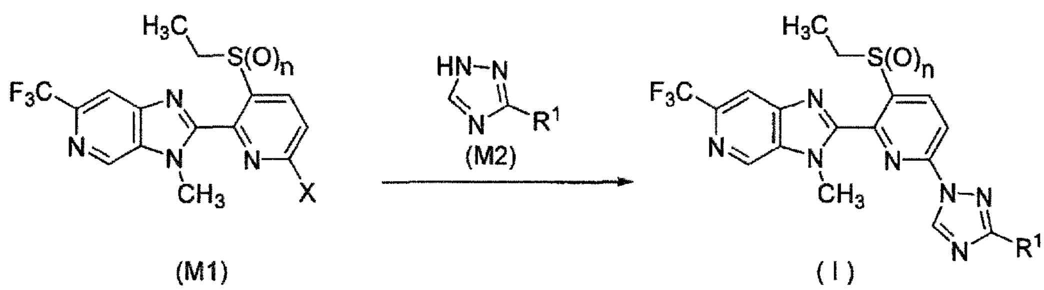

[wherein, X represents a halogen atom, and the other symbols are the same as defined above.]

The compound (M2) is publicly known or may be prepared according to the publicly known method.

The reaction is usually carried out in a solvent. Examples of the solvent to be used in the reaction include ethers such as tetrahydrofuran, ethyleneglycol dimethyl ether, methyl tert-butyl ether and 1,4-dioxane (hereinafter, referred to as "ethers"), halogenated hydrocarbons, aromatic hydrocarbons such as toluene and xylene (hereinafter, referred to as "aromatic hydrocarbons"), esters, nitriles, aprotic polar solvents such as dimethylformamide (hereinafter, referred to as "DMF"), N-methyl pyrrolidone, dimethyl sulfoxide (hereinafter, referred to as "DMSO") (hereinafter, referred to as "aprotic polar solvents"), and nitrogen-containing aromatic solvents such as pyridine and quinoline (hereinafter, referred to as "nitrogen-containing aromatic solvents"), and mixtures thereof.

Examples of the base to be used in the reaction include alkali metal halides such as sodium hydride (hereinafter, referred to as "alkali metal hydrides"), alkali metal carbonates such as sodium carbonate and potassium carbonate (hereinafter, referred to as "alkali metal carbonates"), and organic bases such as triethylamine, diisopropylethylamine, pyridine, 4-(dimethylamino)pyridine (hereinafter, referred to as "organic bases").

In the reaction, the compound (M2) is used usually within a range of 1 to 2 molar ratio(s), the base is used usually within a range of 1 to 5 molar ratio(s), relative to 1 mole of the compound (M1).

The reaction temperature is usually within a range of 0 to 120°C. The reaction period is usually within a range of 0.1 to 24 hours.

When the reaction is completed, water is added to reaction mixtures, and the reaction mixtures are extracted with organic solvent(s), and the organic layers are worked up (for example, drying and concentration) to give the compound (I) of the present invention.

Process 3

In the compound (M1), a compound wherein n = 1 (hereinafter, referred to as "compound (M1b)") and a compound wherein n = 2 (hereinafter, referred to as "compound (M1c)") may be prepared by reacting a compound wherein n = 0 (hereinafter, referred to as "compound (M1a)") with an oxidizing agent.

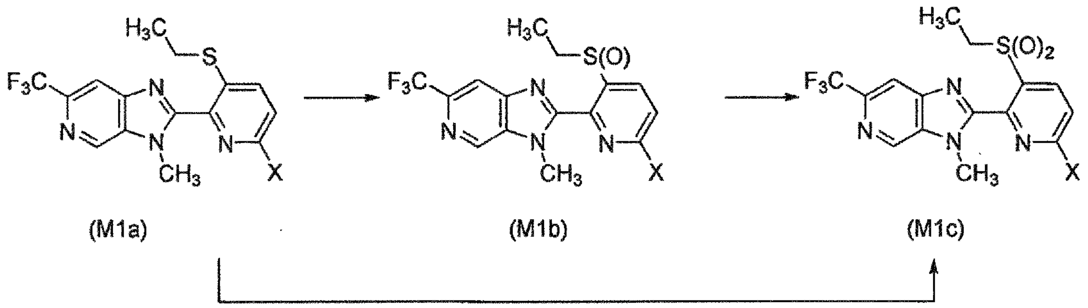

[wherein, the symbols are the same as defined above.]

The reaction may be carried out according to the method of Process 1.

Process 4

The compound (M1a) may be prepared according to the following scheme.

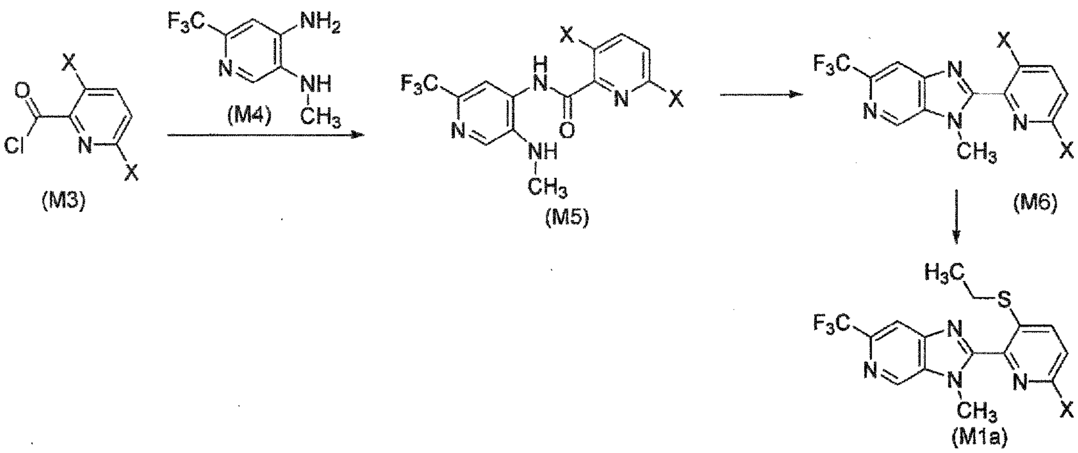

[wherein, the symbols are the same as defined above.]

A compound represented by formula (M5) (hereinafter, referred to as "compound (M5)") may be prepared by reacting a compound represented by formula (M3) (hereinafter, referred to as "compound (M3)") with a compound represented by formula (M4) (hereinafter, referred to as "compound (M4)").

The reaction is usually carried out in a solvent. Examples of the solvent to be used in the reaction include ethers, aliphatic hydrocarbons such as hexane, heptane and octane, aromatic hydrocarbons, halogenated hydrocarbons, esters, nitriles, aprotic polar solvents, and mixtures thereof.

A base may be added to the reaction as needed. Examples of the base to be used in the reaction include alkali metal carbonates and organic bases.

In the reaction, the compound (M4) is used usually within a range of 1 to 3 molar ratio(s), and the base is used usually within a range of 1 to 10 molar ratio(s), relative to 1 mole of the compound (M3).

The reaction temperature is usually within a range of -20 to 100°C. The reaction period is usually within a range of 0.1 to 24 hours.

When the reaction is completed, water is added to reaction mixtures, and the reaction mixtures are extracted with organic solvent(s), and the resulting organic layers are worked up (for example, drying and concentration) to give the compound (M5).

The compound (M3) may be a commercially available compound, or may be prepared by a known method. The compound (M4) may be prepared by the method described in WO 2010/125985.

A compound represented by formula (M6) (hereinafter, referred to as "compound (M6)") may be prepared by an intramolecular-condensation of the compound (M5).

The reaction is usually carried out in a solvent. Examples of the solvent to be used in the reaction include ethers, halogenated hydrocarbons, aromatic hydrocarbons, esters, nitriles such as acetonitrile, aprotic polar solvents, nitrogen-containing aromatic compounds, and mixtures thereof.

In the reaction, a condensing agent, an acid, a base, or a chlorinating agent may be added as needed.

Examples of the condensing agent to be used in the reaction include acetic anhydride, trifluoroacetic anhydride, 1-ethyl-3-(3-dimethylaminopropyl)carbodiimide hydrochloride, a mixture of triphenylphosphine, base(s), carbon tetrachloride or carbon tetrabromide, and a mixture of triphenylphosphine and azodiesters (for example, diethyl azodicarboxylate).

Examples of the acid to be used in the reaction include sulfonic acids such as para-toluene sulfonic acid, and carboxylic acids such as acetic acid, and polyphosphoric acid.

Examples of the base to be used in the reaction include organic bases, alkali metal carbonates, and alkali metal hydrides.

Examples of the chlorinating agent to be used in the reaction include phosphorus oxychloride.

In the reaction, when the condensing agent is used, the condensing agent is used usually within a range of 1 to 5 molar ratio(s), and when the acid is used, the acid is used usually within a range of 0.1 to 5 molar ratio(s), when the base is used, the base is used usually within a range of 1 to 5 molar ratio (s), and when the chlorinating agent is used, the chlorinating agent is used usually within a range of 1 to 5 molar ratio(s), relative to 1 mole of the compound (M5).

The reaction temperature is usually within a range of 0 to 200°C. The reaction period is usually within a range of 0.1 to 24 hours.

When the reaction is completed, water is added to reaction mixtures, and the reaction mixtures are extracted with organic solvent(s), and the resulting organic layers are worked up (for example, drying and concentration) to give the compound (M6).

The compound (M1a) may be prepared by reacting the compound (M6) with ethanethiol in the presence of a base.
The reaction is usually carried out in a solvent. Examples of the solvent to be used in the reaction include ethers, aromatic hydrocarbons, nitriles, aprotic polar solvents, water, and mixtures thereof.
The base to be used in the reaction includes alkali metal carbonates, and alkali metal hydrides.
In the reaction, ethanethiol is usually within a range of 1 to 10 molar ratio(s), and the base is usually used in a range of 1 to 10 molar ratio (s), relative to 1 mole of the compound (M6).
The reaction temperature is usually within a range of -20 to 150°C. The reaction period is usually within a range of 0.5 to 24 hours.
When the reaction is completed, water is added to reaction mixtures, and the reaction mixtures are extracted with organic solvent(s), and the resulting organic layers are worked up (for example, drying and concentration) to give the compound (M1a).

Process 5-1

A compound represented by formula (M8) (hereinafter, referred to as "compound (M8)") may be prepared by reacting a compound represented by formula (M7) (hereinafter, referred to as "compound (M7)") with ethanethiol in the presence of a base.

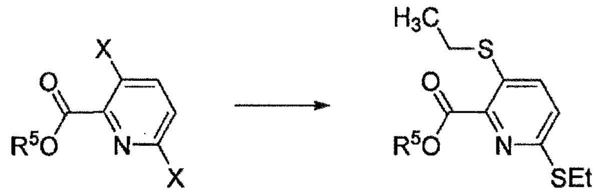

(M7) (M8)

[wherein, $R^5$ represents methyl group or ethyl group, and the other symbols are the same as defined above.]
The reaction may be carried out according to the method described in Process 4 except that ethanethiol is usually used within a range of 2 to 20 molar ratio(s), and the base is usually used in a range of 1 to 10 molar ratio(s), relative to 1 mole of the compound (M7).

Process 5-2

A compound represented by formula (M9) (hereinafter, referred to as "compound (M9)") may be prepared by reacting the compound (M8) with an oxidizing agent.

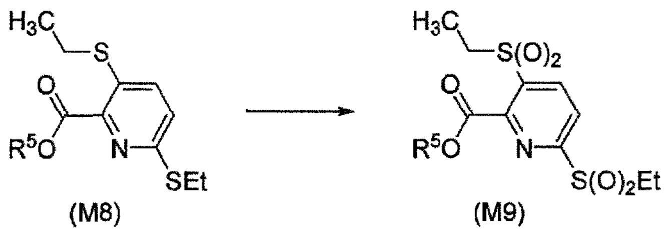

(M8) (M9)

[wherein, the symbols are the same as defined above.]

The reaction may be carried out according to the method described in Process 1 except that the oxidizing agent is usually used within a range of 4 to 5 molar ratios, the base is usually used within a range of 0.04 to 4 molar ratio(s), and the catalyst is usually used within a range of 0.04 to 2 molar ratio(s), relative to 1 mole of the compound (M8).

Process 5-3

A compound represented by formula (M11) (hereinafter, referred to as "compound (M11)") may be prepared by reacting the compound (M9) with a compound represented by formula (M10) (hereinafter, referred to as "compound (M10)").

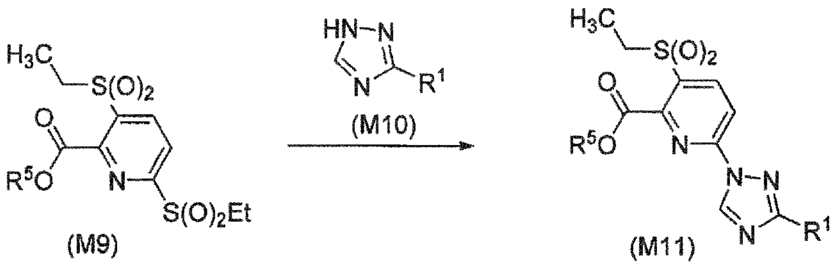

(M9) (M10) (M11)

[wherein, the symbols are the same as defined above.]

The reaction may be carried out according to the method described in Process 2.

Process 5-4

A compound represented by formula (M12) (hereinafter, referred to as "compound (M12)") may be prepared by hydrolyzing the compound (M11) in the presence of a base.

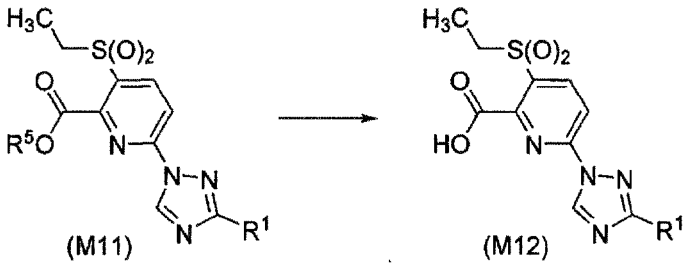

(M11) (M12)

[wherein, the symbols are the same as defined above.]

The reaction is usually carried in a solvent. Examples of the solvent to be used in the reaction include alcohols, water, and mixtures thereof.

The base to be used in the reaction includes alkali metal hydroxides such as sodium hydroxide, and potassium hydroxide.

In the reaction, the base is usually used within a range of 1 to 10 molar ratio (s) relative to 1 mole of the compound (M11).

The reaction temperature is usually within a range of 0 to 100°C. The reaction period is usually within a range of 0.1 to 24 hours.

When the reaction is completed, hydrochloric acid is added to reaction mixtures, and the reaction mixtures are extracted with organic solvent(s), and the resulting organic layers are worked up (for example, drying and concentration) to give the compound (M12).

Process 5-4

A compound represented by formula (M13) (hereinafter, referred to as "compound (M13)") may be prepared by reacting the compound (M12) with the compound (M4) in the presence of a condensing agent and a base.

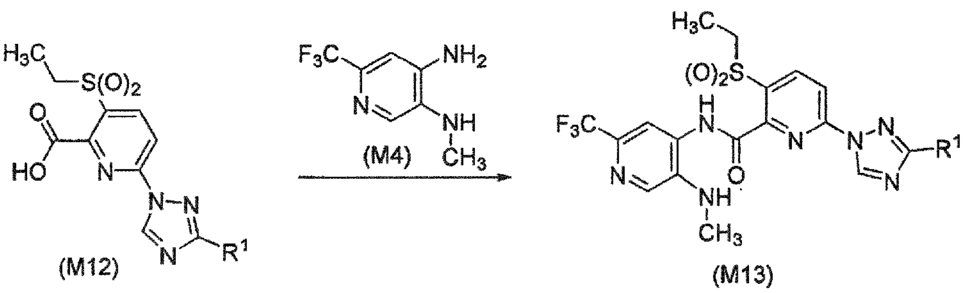

(M12) (M4) (M13)

[wherein, the symbols are the same as defined above.]

The reaction is usually used in a solvent. Examples of the solvent to be used in the reaction include ethers, hydrocarbons, halogenated hydrocarbons, esters, nitriles, aprotic polar solvents, nitrogen-containing aromatic solvents, and mixtures thereof.

Examples of the condensing agent to be used in the reaction include 1H-benzotriazol-1-yloxytris(dimethylamino)phosphonium hexafluorophosphate salt (hereinafter, referred to as "BOP reagent").

The base to be used in the reaction includes alkali metal carbonates, and organic bases.

In the reaction, the compound (M4) is usually used within a range of 1 to 3 molar ratio(s), and the base is usually used within a range of 1 to 10 molar ratio(s), relative to 1 mole of the compound (M12).

The reaction temperature is usually within a range of -20 to 100°C. The reaction period is usually within a range of 0.1 to 24 hours.

When the reaction is completed, water is added to reaction mixtures, and the resulting mixtures are extracted with organic solvent(s), and the resulting organic layers are worked up (for example, drying and concentration) to give the compound (M13).

Process 5-5

The present compound (Ic) may be prepared by an intramolecular-condensation of the compound (M13).

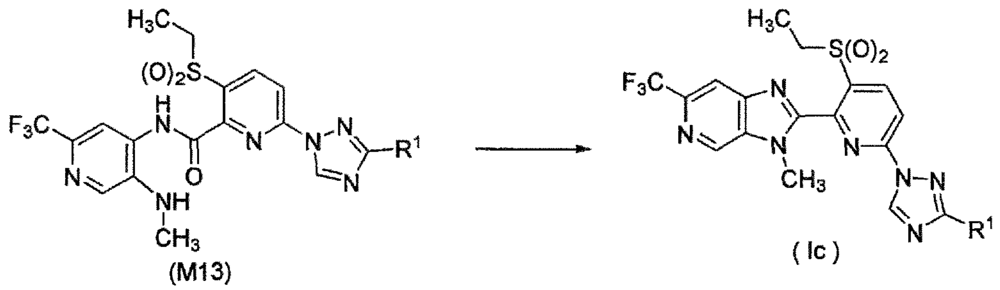

(M13)

(Ic)

[wherein, the symbols are the same as defined above.]

The reaction is usually carried in a solvent. Examples of the solvent to be used in the reaction include ethers, halogenated hydrocarbons, hydrocarbons, esters, nitriles such as acetonitrile, aprotic polar solvents, nitrogen-containing aromatic solvents, and mixtures thereof.

In the reaction, a condensing agent, an acid, a base or a chlorinating agent may be added as needed.

Examples of the condensing agent to be used in the reaction include acetic anhydride, and trifluoroacetic anhydride.

Examples of the acid to be used in the reaction include sulfonic acids such as para-toluene sulfonic acid, carboxylic acids such as acetic acid, and polyphosphoric acid.

Examples of the base to be used in the reaction include organic bases, alkali metal carbonates, and alkali metal hydrides.

Examples of the chlorinating agent to be used in the reaction include phosphorus oxychloride.

In the reaction, when the condensing agent is used, the condensing agent is usually used within a range of 1 to 5 molar ratio (s), and when the acid is used, the acid is usually used within a range of 0.1 to 5 molar ratio(s), and when the base is used, the base is usually used within a range of 1 to 5 molar ratio(s), and when the chlorinating agent is used, the chlorinating agent is usually used within a range of 1 to 5 molar ratio(s), relative to 1 mole of the compound (M13).

The reaction temperature is usually within a range of 0 to 200°C. The reaction period is usually within a range of 0.1 to 24 hours.

When the reaction is completed, water is added to reaction mixtures, and the resulting mixtures are extracted with organic solvent(s), and the resulting organic layers are worked up (for example, drying and concentration) to give the present compound (Ic).

Process 6

A compound represented by formula (1-N) (hereinafter, referred to as "present compound (I-N)") may be prepared according to the following scheme.

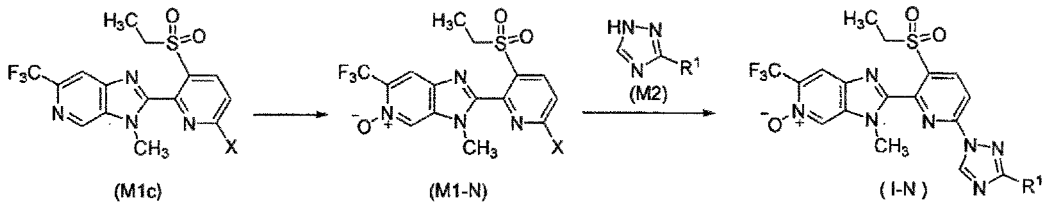

(M1c) (M1-N) (M2) (I-N)

[wherein, the symbols are the same as defined above.]

A compound represented by formula (M1-N) (hereinafter, referred to as "compound (M1-N)") may be prepared by reacting the compound (M1c) with an oxidizing agent.

The reaction is usually carried out in a solvent. Examples of the solvent to be used in the reaction include halogenated hydrocarbons.

Examples of the oxidizing agent to be used in the reaction include mCPBA.

In the reaction, the oxidizing agent is usually used within a range of 1 to 10 molar ratio(s), relative to 1 mole of the compound (M1c).

The reaction temperature is usually within a range of -20 to 80°C. The reaction period is usually within a range of 0.1 to 12 hours.

When the reaction is completed, the reaction mixtures are extracted with organic solvents, and the resulting organic layers are washed with an aqueous solution of a reducing agent (such as sodium sulfite, and sodium thiosulfate), and an aqueous solution of a base (such as sodium hydrogen carbonate) successively. The resulting organic layers are worked up (for example, drying and concentration) to give the compound (M1-N).

The present compound (1-N) may be prepared from the compound (M1-N).

The reaction may be carried out according to the reaction described in Process 2.

Next, specific examples of the present compound are shown below.

A present compound represented by formula (I):

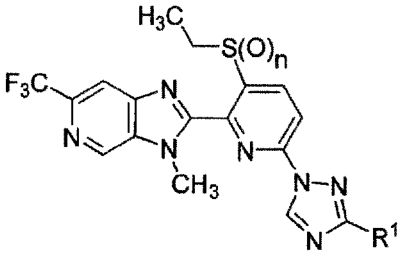

(I)

wherein n and $R^1$ represent any combination indicated in [Table 1] to [Table 3].

[Table 1]

| $R^1$ | n |
|---|---|
| H | 0 |
| H | 1 |
| H | 2 |
| F | 0 |
| F | 1 |
| F | 2 |
| Cl | 0 |
| Cl | 1 |
| Cl | 2 |
| Br | 0 |
| Br | 1 |

(continued)

| $R^1$ | n |
|---|---|
| Br | 2 |
| I | 0 |
| I | 1 |
| I | 2 |
| $CH_3$ | 0 |
| $CH_3$ | 1 |
| $CH_3$ | 2 |
| $CH_2CH_3$ | 0 |
| $CH_2CH_3$ | 1 |
| $CH_2CH_3$ | 2 |
| $CH_2CH_2CH_3$ | 0 |
| $CH_2CH_2CH_3$ | 1 |
| $CH_2CH_2CH_3$ | 2 |
| $CH(CH_3)_2$ | 0 |
| $CH(CH_3)_2$ | 1 |
| $CH(CH_3)_2$ | 2 |
| $CF_3$ | 0 |
| $CF_3$ | 1 |
| $CF_3$ | 2 |
| $CF_2CF_3$ | 0 |
| $CF_2CF_3$ | 1 |
| $CF_2CF_3$ | 2 |
| $CF_2CF_2CF_3$ | 0 |
| $CF_2CF_2CF_3$ | 1 |
| $CF_2CF_2CF_3$ | 2 |
| $CF(CF_3)_2$ | 0 |
| $CF(CF_3)_2$ | 1 |
| $CF(CF_3)_2$ | 2 |
| $CH_2CF_3$ | 0 |
| $CH_2CF_3$ | 1 |
| $CH_2CF_3$ | 2 |
| $OCH_3$ | 0 |
| $OCH_3$ | 1 |
| $OCH_3$ | 2 |
| $OCH_2CH_3$ | 0 |
| $OCH_2CH_3$ | 1 |
| $OCH_2CH_3$ | 2 |
| $OCH_2CH_2CH_3$ | 0 |

(continued)

| $R^1$ | n |
|---|---|
| $OCH_2CH_2CH_3$ | 1 |
| $OCH_2CH_2CH_3$ | 2 |
| $OCH(CH_3)_2$ | 0 |
| $OCH(CH_3)_2$ | 1 |
| $OCH(CH_3)_2$ | 2 |

[Table 2]

| $R^1$ | n |
|---|---|
| $SCH_3$ | 0 |
| $SCH_3$ | 1 |
| $SCH_3$ | 2 |
| $SCH_2CH_3$ | 0 |
| $SCH_2CH_3$ | 1 |
| $SCH_2CH_3$ | 2 |
| $SCH_2CH_2CH_3$ | 0 |
| $SCH_2CH_2CH_3$ | 1 |
| $SCH_2CH_2CH_3$ | 2 |
| $SCH(CH_3)_2$ | 0 |
| $SCH(CH_3)_2$ | 1 |
| $SCH(CH_3)_2$ | 2 |
| $S(O)CH_3$ | 0 |
| $S(O)CH_3$ | 1 |
| $S(O)CH_3$ | 2 |
| $S(O)CH_2CH_3$ | 0 |
| $S(O)CH_2CH_3$ | 1 |
| $S(O)CH_2CH_3$ | 2 |
| $S(O)CH_2CH_2CH_3$ | 0 |
| $S(O)CH_2CH_2CH_3$ | 1 |
| $S(O)CH_2CH_2CH_3$ | 2 |
| $S(O)CH(CH_3)_2$ | 0 |
| $S(O)CH(CH_3)_2$ | 1 |
| $S(O)CH(CH_3)_2$ | 2 |
| $S(O)_2CH_3$ | 0 |
| $S(O)_2CH_3$ | 1 |
| $S(O)_2CH_3$ | 2 |
| $S(O)_2CH_2CH_3$ | 0 |
| $S(O)_2CH_2CH_3$ | 1 |

(continued)

| $R^1$ | n |
|---|---|
| $S(O)_2CH_2CH_3$ | 2 |
| $S(O)_2CH_2CH_2CH_3$ | 0 |
| $S(O)_2CH_2CH_2CH_3$ | 1 |
| $S(O)_2CH_2CH_2CH_3$ | 2 |
| $S(O)_2CH(CH_3)_2$ | 0 |
| $S(O)_2CH(CH_3)_2$ | 1 |
| $S(O)_2CH(CH_3)_2$ | 2 |
| $NH_2$ | 0 |
| $NH_2$ | 1 |
| $NH_2$ | 2 |
| $NHCH_3$ | 0 |
| $NHCH_3$ | 1 |
| $NHCH_3$ | 2 |
| $NHCH_2CH_3$ | 0 |
| $NHCH_2CH_3$ | 1 |
| $NHCH_2CH_3$ | 2 |
| $NHCH_2CH_2CH_3$ | 0 |
| $NHCH_2CH_2CH_3$ | 1 |
| $NHCH_2CH_2CH_3$ | 2 |
| $NHCH(CH_3)_2$ | 0 |
| $NHCH(CH_3)_2$ | 1 |
| $NHCH(CH_3)_2$ | 2 |
| $N(CH_3)_2$ | 0 |
| $N(CH_3)_2$ | 1 |
| $N(CH_3)_2$ | 2 |

[Table 3]

| $R^1$ | n |
|---|---|
| $N(CH_2CH_3)_2$ | 0 |
| $N(CH_2CH_3)_2$ | 1 |
| $N(CH_2CH_3)_2$ | 2 |
| $N(CH_2CH_2CH_3)_2$ | 0 |
| $N(CH_2CH_2CH_3)_2$ | 1 |
| $N(CH_2CH_2CH_3)_2$ | 2 |
| $N[CH(CH_3)_2]_2$ | 0 |
| $N[CH(CH_3)_2]_2$ | 1 |
| $N[CH(CH_3)_2]_2$ | 2 |

(continued)

| $R^1$ | n |
|---|---|
| $N(CH_3)CH_2CH_3$ | 0 |
| $N(CH_3)CH_2CH_3$ | 1 |
| $N(CH_3)CH_2CH_3$ | 2 |
| $N(CH_3)CH_2CH_2CH_3$ | 0 |
| $N(CH_3)CH_2CH_2CH_3$ | 1 |
| $N(CH_3)CH_2CH_2CH_3$ | 2 |
| $N(CH_3)CH(CH_3)_2$ | 0 |
| $N(CH_3)CH(CH_3)_2$ | 1 |
| $N(CH_3)CH(CH_3)_2$ | 2 |
| $N(CH_2CH_3)CH_2CH_2CH_3$ | 0 |
| $N(CH_2CH_3)CH_2CH_2CH_3$ | 1 |
| $N(CH_2CH_3)CH_2CH_2CH_3$ | 2 |
| $N(CH_2CH_3)CH(CH_3)_2$ | 0 |
| $N(CH_2CH_3)CH(CH_3)_2$ | 1 |
| $N(CH_2CH_3)CH(CH_3)_2$ | 2 |
| $C(O)OCH_3$ | 0 |
| $C(O)OCH_3$ | 1 |
| $C(O)OCH_3$ | 2 |
| $C(O)OCH_2CH_3$ | 0 |
| $C(O)OCH_2CH_3$ | 1 |
| $C(O)OCH_2CH_3$ | 2 |
| $C(O)OCH_2CH_2CH_3$ | 0 |
| $C(O)OCH_2CH_2CH_3$ | 1 |
| $C(O)OCH_2CH_2CH_3$ | 2 |
| $C(O)OCH(CH_3)_2$ | 0 |
| $C(O)OCH(CH_3)_2$ | 1 |
| $C(O)OCH(CH_3)_2$ | 2 |
| $NO_2$ | 0 |
| $NO_2$ | 1 |
| $NO_2$ | 2 |
| CN | 0 |
| CN | 1 |
| CN | 2 |

A present compound represented by formula (I-N):

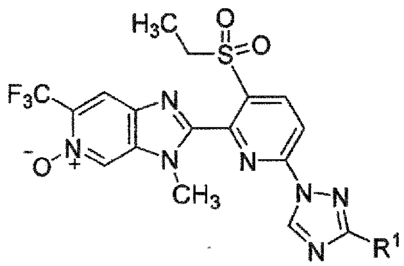

( I-N )

wherein $R^1$ represents a group indicated in [Table 4].

[Table 4]

| $R^1$ |
|---|
| H |
| F |
| Cl |
| Br |
| I |
| $CH_3$ |
| $CH_2CH_3$ |
| $CH_2CH_2CH_3$ |
| $CH(CH_3)_2$ |
| $CF_3$ |
| $CF_2CF_3$ |
| $CF_2CF_2CF_3$ |
| $CF(CF_3)_2$ |
| $CH_2CF_3$ |
| $OCH_3$ |
| $OCH_2CH_3$ |
| $OCH_2CH_2CH_3$ |
| $OCH(CH_3)_2$ |
| $SCH_3$ |
| $SCH_2CH_3$ |
| $SCH_2CH_2CH_3$ |
| $SCH(CH_3)_2$ |
| $S(O)CH_3$ |
| $S(O)CH_2CH_3$ |
| $S(O)CH_2CH_2CH_3$ |
| $S(O)CH(CH_3)_2$ |
| $S(O)_2CH_3$ |
| $S(O)_2CH_2CH_3$ |
| $S(O)_2CH_2CH_2CH_3$ |
| $S(O)_2CH(CH_3)_2$ |

(continued)

| $R^1$ |
|---|
| $NH_2$ |
| $NHCH_3$ |
| $NHCH_2CH_3$ |
| $NHCH_2CH_2CH_3$ |
| $NHCH(CH_3)_2$ |
| $N(CH_3)_2$ |
| $N(CH_2CH_3)_2$ |
| $N(CH_2CH_2CH_3)_2$ |
| $N[CH(CH_3)_2]_2$ |
| $N(CH_3)CH_2CH_3$ |
| $N(CH_3)CH_2CH_2CH_3$ |
| $N(CH_3)CH(CH_3)_2$ |
| $N(CH_2CH_3)CH_2CH_2CH_3$ |
| $N(CH_2CH_3)CH(CH_3)_2$ |
| $C(O)OCH_3$ |
| $C(O)OCH_2CH_3$ |
| $C(O)OCH_2CH_2CH_3$ |
| $C(O)OCH(CH_3)_2$ |
| $NO_2$ |
| CN |

Examples of harmful arthropod pests against which the present compound has an efficacy include harmful insects and harmful mites. Examples of harmful arthropod pests include the followings.

Hemiptera:

Delphacidae such as *Laodelphax striatellus, Nilaparvata lugens, Sogatella furcifera,* and *Peregrinus maidis;*
Deltocephalidae such as *Nephotettix cincticeps, Nephotettix virescens, Nephotettix nigropictusr* (Rice green leafhopper), *Recilia dorsalis, Empoasca onukii, Empoasca fabae, Dalbulus maidis, Mahanarva posticata* (Sugarcane froghopper), *Mahanarva fimbriolota* (Sugarcane root spittlebug), *Cofana spectra, Nephotettix nigropictus,* and *Recilia dorsalis;*
Aphididae such as *Aphis gossypii, Myzus persicae, Brevicoryne brassicae, Aphis spiraecola, Macrosiphum euphorbiae, Aulacorthum solani, Rhopalosiphum padi, Toxoptera citricidus, Hyalopterus pruni, Aphis glycines Matsumura, Rhopalosiphum maidis, Tetraneura nigriabdominalis, Viteus vitifoliae, Daktulosphaira vitifoliae* (Grape Phylloxera), *Phylloxera devastatrix Pergande* (Pecan phylloxera), *Phylloxera notabilis pergande* (Pecan leaf phylloxera), and *Phylloxera russellae Stoetzel* (Southern pecan leaf phylloxera);
Pentatomidae such as *Scotinophara lurida, Scotinophara coarctata* (Malayan rice black bug), *Nezara antennata, Eysarcoris parvus, Halyomorpha mista, Nezara viridula, Euschistus heros* (Brown stink bug), *Nezara viridula* (Southern green stink bug), *Piezodorus guildinii* (Red banded stink bug), *Scaptocoris castanea* (Burrower brown bug), *Oebalus pugnax,* and *Dichelops melacanthus;*
Alydidae such as *Riptortus clavetus, Leptocorisa chinensis, Leptocorisa acuta,* or *Leptocorisa* spp.;
Miridae such as *Trigonotylus caelestialium, Stenotus rubrovittatus, Lygus lineolaris,* and *Blissus leucopterus leucopterus* (Chinchi bug);
Aleyrodidae such as *Trialeurodes vaporariorum, Bemisia tabaci, Dialeurodes citri,* and *Aleurocanthus spiniferus;*
Coccoidea such as *Aonidiella aurantii, Comstockaspis perniciosa, Unaspis citri, Ceroplastes rubens, Icerya purchasi, Planococcus Kraunhiae, Pseudococcus longispinis, Pseudaulacaspis Pentagona,* and *Brevennia rehi;*

Psyllidae such as *Diaphorina citri, Psylla pyrisuga, Bactericerca cockerelli;*
Tingidae such as *Stephanitis nasi;*
Cimicoidea such as *Cimex lectularius;*
*Quesada gigas* (Giant Cicada);
and the others.

Lepidoptera pests:

Pyralidae such as *Chilo suppressalis, Chilo polychrysus* (Darkheaded stm borer), *Tryporyza incertulas, Chilo polychrysus,* Scirpophaga innotata, *Scirpophaga incertulas* (Yellow stem borer), *Sesamia inferens* (Pink borer), *Rupela albinella, Cnaphalocrocis medinalis, Marasmia patnalis, Marasmia exigna, Notarcha derogata, Plodia interpunctella, Ostrinia furnacalis, Hellula undalis, Pediasia teterrellus, Nymphula depunctalis, Marasmia* spp., *Hydraecia immanis* (Hop vine borer), *Ostrinia nubilalis* (European corn borer), *Elasmopalpus lignosellus* (Lesser cornstalk borer), *Epinotia aporema* (Bean Shoot Borer), *Diatraea saccharalis* (Sugarcane borer), and *Telchin licus* (Giant Sugarcane borer);
Noctuidae suh as *Spodoptera litura, Spodoptera exigua, Pseudaletia separata, Mamestra brassicae, Sesamia inferens, Spodoptera mauritia, Spodoptera frugiperda, Spodoptera exempta, Agrotis ipsilon, Plusia nigrisigna, Pseudoplusia includens* (Soybean looper), *Trichoplusia* spp., *Heliothis* spp. (for example, *Heliothis virescens*), *Helicoverpa* spp. (for example, *Helicoverpa armigera*), *Anticarsia gammatalis* (Velvetbean caterpillar), and *Alabama argillacea* (Cotton leafworm));
Pieridae such as *Pieris rapae;*
Adokisofiesu genus;
Tortricidae such as *Grapholita molesta, Leguminivora glycinivorella, Matsumuraeses azukivora, Adoxophyes orana fasciata, Adoxophyes honmai, Homona magnanima, Archips fuscocupreanus,* and *Cydia pomonella;*
Gracillariidae such as *Caloptilia theivora,* and *Phyllonorycter ringoneella;*
Carposinidae such as *Carposina niponensis,* and *Ecdytolopha aurantiana* (Citrus fruit borer);
Lyonetiidae such as *Leucoptera coffeela* (Coffee Leaf miner) and *Lyonetia* spp.;
Lymantriidae such as *Lymantria* spp., and *Euproctis* spp.;
Yponomeutidae such as *Plutella xylostella;*
Gelechiidae such as *Pectinophora gossypiella,* and *Phthorimaea operculella;*
Arctiidae such as *Hyphantria cunea;*
and the others.

Thysanoptera pests:

Thysanopterae such as *Frankliniella occidentalis, Thrips parmi, Scirtothrips dorsalis, Thrips tabaci, Frankliniella intonsa, Frankliniella occidentalis, Haplothrips aculeatus,* and *Stenchaetothrips biformis;*
and the others.

Diptera pests:

Diptera:

House mosquitoes (Culex spp.) such as *Culex pipiens pallens, Culex tritaeniorhynchus,* and *Culex quinquefasciatus;*
Aedes spp. such as *Aedes aegypti,* and *Aedes albopictus;*
Anopheles spp. such as Anopheles sinensis;
Chironomidae;
Muscidae such as *Musca domestica,* and *Muscina stabulans;*
Anthomyiidae such as *Delia platura, Delia antiqua,* and *Tetanops myopaeformis;*
Agromyzidae such as *Agromyza oryzae, Hydrellia griseola, Liriomyza sativae, Liriomyza trifolii,* and *Chromatomyia horticola;*
Chloropidae such as *Chlorops oryzae;*
Tephritidae such as *Dacus cucurbitae,* and *Ceratitis capitata;*
Ephydridae such as *Hydrellia philippina,* and *Hydrellia sasakii;*
Drosophilidae;
Phoridae such as *Megaselia spiracularis;*
Psychodidae such as *Clogmia albipunctata;*
Sciaridae;

Cecidomyiidae such as *Mayetiola destructor,* and *Orseolia oryzae;*
Diopsidae such as *Diopsis macrophthalma;*
Tipulidae such as *Tipula oleracea* (Common cranefly), and *Tipula paludosa* (European cranefly);
and the others.

Chrysomelidae

*Diabrotica virgifera virgifera, Diabrotica undecimpunctata howardi, Diabrotica barberi,* Diabrotica virgifera zeae, *Diabrotica balteata LeConte, Diabrotica speciosa, Diabrotica speciosa* (Cucurbit Beetle), *Cerotoma trifurcata, Oulema melanopus, Aulacophora femoralis, Phyllotreta striolata, Leptinotarsa decemlineata, Oulema oryzae, Colaspis brunnea, Chaetocnema pulicaria, Epitrix cucumeris, Dicladispa armigera, Stenolophus lecontei* (Seedcorn beetle), and *Clivinia impressifrons* (Slender seedcorn beetle));
Scarabaeidae such as Anomala cuprea, *Anomala rufocuprea, Popillia japonica, Rhizotrogus majalis* (European Chafer), *Bothynus gibbosus* (Carrot beetle), *Colaspis brunnea* (Grape Colaspis), *Myochrous denticollis* (southern Corn leaf beetle), *Holotrichia* spp., and *Phyllophaga* spp. (for example, Phyllophaga crinita));
Erirhinidae such as *Sitophilus zeamais, Echinocnemus squameus, Lissorhoptrus oryzophilus,* and *Sphenophorus venatus;*
Curculionidae such as *Anthonomus grandis, Sphenophorus callosus* (Southern Corn Billbug), *Sternechus subsignatus* (Soybean stalk weevil), and *Sphenophorus* spp. (for example, Sphenophorus levis);
Epilachna such as *Epilachna vigintioctopunctata;*
Scolytidae such as *Lyctus brunneus,* and *Tomicus piniperda;*
Bostrichidae;
Ptinidae;
Cerambycidae such as *Anoplophora malasiaca,* and *Migdolus fryanus;*
Elateridae (*Agriotes* sp., *Aelous* sp., *Anchastus* sp., *Melanotus* sp., *Limonius* sp., *Conoderus* sp., *Ctenicera* sp.) such as Melanotus okinawensis, Agriotes ogurae fuscicollis, and Melanotus legatus;
Staphylinidae such as *Paederus fuscipes;*
Hypothenemus hampei (Coffee Barry Borer);
and the others.

Orthoptera pests:

*Locusta migratoria, Gryllotalpa africana, Dociostaurus maroccanus, Chortoicetes terminifera, Nomadacris septemfasciata, Locustana pardalina* (Brown Locust), *Anacridium melanorhodon* (Tree Locust), *Calliptamus italicus* (Italian Locust), *Melanoplus differentialis* (Differential grasshopper), *Melanoplus bivittatus* (Twostriped grasshopper), *Melanoplus sanguinipes* (Migratory grasshopper), *Melanoplus femurrubrum* (Red-Legged grasshopper), *Camnula pellucida* (Clearwinged grasshopper), *Schistocerca gregaria, Gastrimargus musicus* (Yellow-winged locust), *Austracris guttulosa* (Spur-throated locust), *Oxya yezoensis, Oxya japonica, Patanga succincta,* and *Grylloidea* (such as *Acheta domesticus, Teleogryllus emma,* and *Anabrus simplex* (Mormon cricket));
and the others.

Hymenoptera pests:

*Tenthredinidae* such as *Athalia rosae,* and Athalia *japonica;*
*Solenopsis* spp.;
*Acromyrmex* spp. such as *Atta capiguara* (Brown leaf-cutting ant));
and the others.

Blattariae pests:

*Blattella germanica, Periplaneta fuliginosa, Periplaneta americana, Periplaneta brunnea, Blatta orientalis,* and the others.

Isoptera pests:

*Reticulitermes speratus, Coptotermes formosanus, Incisitermes minor, Cryptotermes domesticus, Odontotermes formosanus, Neotermes koshunensis, Glyptotermes satsumensis, Glyptotermes nakajimai, Glyptotermes fuscus, Glyptotermes kodamai, Glyptotermes kushimensis, Hodotermopsis sjostedti, Coptotermes guangzhoensis, Reticuli-*

*termes amamianus, Reticulitermes miyatakei, Reticulitermes kanmonensis, Nasutitermes takasagoensis, Pericapritermes nitobei, Sinocapritermes mushae,* and *Cornitermes cumulans;*
and the others.

Acarina pests:

Tetranychidae such as *Tetranychus urticae, Tetranychus kanzawai, Panonychus citri, Panonychus ulmi, Oligonychus* spp., and *Brevipalpus phoenicis* (Southern Turkey spider mites)),
Eriophyidae such as *Aculops pelekassi, Phyllocoptruta citri, Aculops lycopersici, Calacarus carinatus, Acaphylla theavagrans, Eriophyes chibaensis,* and *Aculus schlechtendali;*
Tarsonemidae such as *Polyphagotarsonemus latus;*
Tenuipalpidae such as *Brevipalpus phoenicis;*
Tuckerellidae;
Ixodidae such as *Haemaphysalis longicornis, Haemaphysalis flava, Dermacentor taiwanicus, Dermacentor variabilis, Ixodes ovatus, Ixodes persulcatus, Ixodes scapularis, Amblyomma americanum, Boophilus microplus,* and *Rhipicephalus sanguineus;*
Acaridae such as *Tyrophagus* putrescentiae, and *Tyrophagus similis;*
Pyroglyphidae such as *Dermatophagoides farinae,* and *Dermatophagoides ptrenyssnus;*
and the others.

The agent for controlling harmful arthropod pests of the present invention comprises the present compound and an inert active carrier. The agent for controlling harmful arthropod pests is usually prepared by mixing the present compound with an inert active carrier such as solid carrier, liquid carrier or gaseous carrier, and if necessary, adding surfactants and the other auxiliary agents for formulation, to formulate into emulsifiable concentrates, oil solutions, dust formulations, granules, wettable powders, flowables, microcapsules, aerosols, smoking agents, poison baits, resin formulations, shampoo formulations, paste-like formulations, foams, carbon dioxide formulations and tablets, and the others.
Such formulations may be processed into mosquito repellent coils, electric mosquito repellent mats, liquid mosquito formulations, smoking agents, fumigants, sheet formulations, spot-on formulations or formulations for oral treatment.
Such formulations may be processed into mosquito repellent coils, electric mosquito repellent mats, liquid mosquito formulations, smoking agents, fumigants, sheet formulations, spot-on formulations or formulations for oral treatment.
Also, the agent for controlling harmful arthropod pests of the present invention may be mixed with other pesticides, miticides, nematicides, fungicides, plant growth regulators, herbicides, and synergists.

The agent for controlling harmful arthropod pests of the present invention comprises usually 0.01 to 95% by weight of the present compound.

Examples of the solid carrier to be used in the formulation include fine powders or granules of clays (for example, kaolin clay, diatomaceous earth, bentonite, Fubasami clay, or acid white clay), synthetic hydrated silicon oxides, talcs, ceramics, other inorganic minerals (for example, sericite, quartz, sulfur, active carbon, calcium carbonate or hydrated silica) and chemical fertilizers (for example, ammonium sulfate, ammonium phosphate, ammonium nitrate, urea or ammonium chloride) and the others; as well as synthetic resins (for example, polyester resins such as polypropylene, polyacrylonitrile, polymethylmethacrylate and polyethylene terephthalate; nylon resins (for example, nylon-6, nylon-11 and nylon-66); polyamide resins; polyvinyl chloride, polyvinylidene chloride, vinyl chloride-propylene copolymers, and the others).

Examples of the above-mentioned liquid carriers include water; alcohols (for example, methanol, ethanol, isopropyl alcohol, butanol, hexanol, benzyl alcohol, ethylene glycol, propylene glycol or phenoxy ethanol); ketones (for example, acetone, methyl ethyl ketone or cyclohexanone); aromatic hydrocarbons (for example, toluene, xylene, ethyl benzene, dodecyl benzene, phenyl xylyl ethane or methylnaphthalene); aliphatic hydrocarbons (for example, hexane, cyclohexane, kerosene or light oil); esters (for example, ethyl acetate, butyl acetate, isopropyl myristate, ethyl oleate, diisopropyl adipate, diisobutyl adipate or propylene glycol monomethyl ether acetate); nitriles (for example, acetonitrile or isobutyronitrile); ethers (for example, diisopropyl ether, 1,4-dioxane, ethyleneglycol dimethyl ether, diethyleneglycol dimethyl ether, diethylene glycol monomethyl ether, propylene glycol monomethyl ether, dipropylene glycol monomethyl ether, or 3-methoxy-3-methyl-1-butanol); acid amides (for example, DMF, N,N-dimethylacetamide); dimethyl sulfoxide (DMSO); propylene carbonate; and vegetable oils (for example, soybean oil or cottonseed oil).

Examples of the above-mentioned gaseous carrier include fluorocarbon, butane gas, LPG (liquefied petroleum gas), dimethyl ether, and carbon dioxide gas.

Examples of the surfactants include nonionic surfactants such as polyoxyethylenated alkyl ethers, polyoxyethylenated alkyl aryl ethers, and polyethylene glycol fatty acid esters; and anionic surfactants such as alkyl sulfonates, alkylbenzene sulfonates, and alkyl sulfates.

Examples of the other auxiliary agents for formulation include a binder, a dispersant, a colorant and a stabilizer. Specific examples include casein, gelatin, polysaccharides (for example, starch, gum arabic, cellulose derivatives and

alginic acid), lignin derivatives, bentonite, water-soluble synthetic polymers (for example, polyvinyl alcohol, polyvinyl pyrrolidone, or polyacrylic acids), PAP (acidic isopropyl phosphate), BHT (2,6-di-tert-butyl-4-methylphenol), BHA (a mixture of 2-tert-butyl-4-methoxyphenol and 3-tert-butyl-4-methoxyphenol).

Examples of base material of the resin formulation include polyvinyl chloride polymers, polyurethane and the others, and a plasticizer such as phthalate esters (for example, dimethyl phthalate, or dioctyl phthalate), adipic acid esters and stearic acid may be added to these base materials, if necessary. The resin formulation can be prepared by kneading the compound of the present invention with the above-mentioned base material in a usual kneading apparatus, followed by molding the mixtures by injection molding, extrusion molding, pressure molding, or the like. The resultant resin formulation can be subjected to further molding or cutting procedure and the like, if necessary, to be processed into shapes such as a plate, film, tape, net or string shape. These resin formulations can be processed into animal collars, animal ear tags, sheet products, trap strings, gardening supports and other products.

Examples of a base material for the poison baits include bait ingredients such as grain powder, vegetable oil, saccharide and crystalline cellulose, and if necessary, with addition of antioxidants such as dibutylhydroxytoluene and nordihydroguaiaretic acid, preservatives such as dehydroacetic acid, accidental ingestion inhibitors for children and pets such as a chili powder, insect attraction fragrances such as cheese flavor, onion flavor and peanut oil.

The method for controlling harmful arthropod pests of the present invention is conducted by applying an effective amount of the present compound to a harmful arthropod pest directly and/or a habitat where the pest lives (for example, plant bodies, soil, an interior of a house, animal bodies). In the method for controlling harmful arthropod pests of the present invention, the present compound is usually used in the form of a harmful arthropod pest controlling agent.

When an agent for controlling harmful arthropod pests of the present invention is used for controlling harmful arthropod pests in an agricultural field, the application dose as an amount of the present compound is usually within a range from 1 to 10,000 g per 10,000 $m^2$. When the agent for controlling harmful arthropod pests is formulated into the emulsifiable concentrate, the wettable powder, or the flowable formulation etc., the agent for controlling harmful arthropod pests of the present invention is usually applied by diluting it with water in such a way that a concentration of the active ingredient is within a range from 0.01 to 10,000 ppm. The granular formulation, or the dust formulation etc., is usually applied as itself without diluting it.

These formulations or a water dilution thereof can be sparged directly to harmful arthropod pests or plants to be protected from harmful arthropod pests, and also may be applied to the soil of crop land in order to control harmful arthropod pests which live there.

The resin preparation which is processed into a sheet or a string may be applied by winding a crop with a sheet or a string of the resin preparation, putting a string of the resin preparation around a crop so that the crop is surrounded by the string, or laying a sheet of the resin preparation on the soil surface near the root of a crop.

When the agent for controlling harmful arthropod pests of the present invention is used to control pests that live inside a house, the application dose as an amount of the present compound is usually within a range from 0.01 to 1,000 mg per 1 $m^2$ of an area to be treated, in the case of using it on a planar area. In the case of using it spatially, the application dose as an amount of the present compound is usually within a range from 0.01 to 500 mg per 1 $m^3$ of the space to be treated. When the agent for controlling harmful arthropod pests of the present invention is formulated into emulsifiable concentrates, wettable powders, flowables or the others, such formulations are usually applied after diluting them with water in such a way that a concentration of the active ingredient is within a range from 0.1 to 10,000 ppm, and then sparging them. In the case of being formulated into oil solutions, aerosols, smoking agents, poison baits and the others, such formulations are applied as itself without diluting it.

When the agent for controlling harmful arthropod pests of the present invention is sued for controlling external parasites of livestock such as cows, horses, pigs, sheep, goats and chickens and small animals such as dogs, cats, rats and mice, the harmful arthropod pest control agent of the present invention can be applied to the animals by a known method in the veterinary field. Specifically, when systemic control is intended, the pest control agent of the present invention is administered to the animals as a tablet, a mixture with feed or a suppository, or by injection (including intramuscular, subcutaneous, intravenous and intraperitoneal injections). On the other hand, when non-systemic control is intended, the pest control agent of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of administering to an animal body, the dose of the present compound is usually within a range from 0.1 to 1,000 mg per 1 kg of an animal body weight.

EXAMPLES

Hereinafter, the present invention is explained in more detail by using Preparation Exmaple, Formulation Example, and Test Example, however, the present invention should not be limited to these examples.

First, with respect to the preparation of the present compound, the Preparation Example is shown.

Preparation Example 1-1

To a mixture of methyl 3,6-dichloropyridine-2-carboxylate 3.0 g, ethanethiol 2.27 mL, and THF 29 mL was added sodium hydride (60%, oily) 1.28 g under ice-cooling. The mixtures were stirred at room temperature for 1 hour, and to the mixtures was added saturated aqueous ammonium chloride solution, and the resulting mixtures were extracted with ethyl acetate. The organic layers were dried over anhydrous sodium sulfate and concentrated. The residues were subjected to a silica gel column chromatography to give an intermediate compound A represented by the following formula 2.80 g.

Intermediate compound A

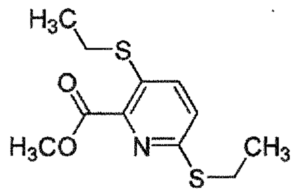

$^1$H-NMR ($CDCl_3$) δ: 7.53 (1H, d), 7.22 (1H, d), 3.97 (3H, s), 3.19 (2H, q), 2.90 (2H, q), 1.38 (3H, t), 1.34 (3H, t).

Preparation Example 1-2

To a mixture of the intermediate compound A 2.80 g, and chloroform 36 mL was added mCPBA 10.28g under ice-cooling, and the mixtures were stirred at room temperature for 8 hours. To the mixtures were added saturated aqueous sodium thiosulfate solution and saturated sodium hydrocarbonate solution successively, and the resulting mixtures were extracted with ethyl acetate. The organic layers were dried over anhydrous sodium sulfate and concentrated to give an intermediate compound B represented by the following formula 3.43 g.

Intermediate compound B

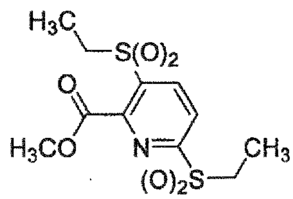

$^1$H-NMR ($CDCl_3$) δ: 8.63 (1H, d), 8.36 (1H, d), 4.05 (3H, s), 3.55 (2H, q), 3.52 (2H, q) 1.38 (3H, t), 1.37 (3H, t).

Preparation Example 1-3

To a mixture of the intermediate compound B 1.0 g and DMF 10 mL were added 1H-1,2,4-triazol 237 mg and potassium carbonate 473 mg, and the mixtures were stirred at room temperature for 5 hours. To the mixtures was added water, and the resulting mixtures were extracted with ethyl acetate. The organic layers were dried over anhydrous sodium sulfate, and concentrated to give an intermediate compound C represented by the following formula 790 mg. Intermediate compound C

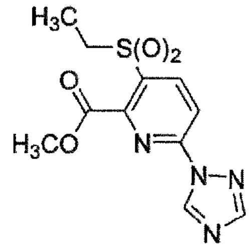

$^1$H-NMR ($CDCl_3$) δ: 9.24 (1H, s), 8.51 (1H, d), 8.18-8.17 (2H, m), 4.07 (3H, s), 3.51 (2H, q), 1.37 (3H, t)

Preparation Example 1-4

To a mixture of the intermediate compound C 790 mg, water 6 mL, and THF 10 mL was added lithium hydroxide 128 mg at room temperature, and the mixtures were stirred at room temperature for 3 hours. To the mixtures was added 1N hydrochloric acid, and the resulting mixtures were extracted with ethyl acetate. The organic layers were dried over anhydrous sodium sulfate, and concentrated to give an intermediate compound D represented by the following formula 473 mg.

Intermediate compound D

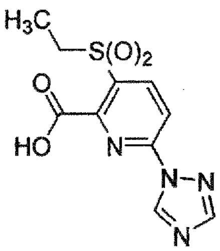

$^1$H-NMR ($CDCl_3$) δ: 9.42 (1H, s), 8.56 (1H, d), 8.23 (1H, s), 8.18 (1H, d), 3.62 (2H, q), 1.36 (3H, t).

Preparation Example 1-5

To a mixture of $N^5$-methyl-2-(trifluoromethyl)pyridine-4,5-diamine 0.50 g, the intermediate compound D 0.81 g, triethylamine 0.48 g, and DMF 10 mL was added BOP reagent 1.27 g, and the mixtures were stirred at room temperature for 1 day. To the mixtures was added water, and the resulting mixtures were extracted with ethyl acetate. The organic layers were washed with saturated brine, dried over anhydrous sodium sulfate, and concentrated. To the residue was added water, and the resulting mixtures were stirred at 100°C for 4 hours. To the reaction solutions was added ice water, and the resulting mixtures were extracted with ethyl acetate. The organic layers were washed with water and saturated brine successively, and dried over anhydrous sodium sulfate, and concentrated. The resulting residues were subjected to a column chromatography to give the present compound 1 represented by the following formula 287 mg.

Present compound 1

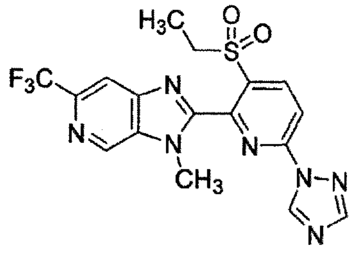

$^1$H-NMR ($CDCl_3$) δ: 9.15 (1H, s), 9.04 (1H, s), 8.71 (1H, d), 8.33 (1H, d), 8.21 (1H, s), 8.15 (1H, d), 3.94 (3H, s), 3.74 (2H, q), 1.38 (3H, t).

Preparation Example 2

To a mixture of 2-[6-chloro-3-(ethansulfonyl)-pyridin-2-yl]-3-methyl-6-(trifluoromethyl)-3H-imidazo[4,5-c]pyridine (prepared by the method described in WO 2016/116338) 0.30 g, cesium carbonate 0.48 g, and DMF 4 mL was added 3-chloro-1H-[1,2,4]triazole 0.08 g, and the mixtures were stirred at 80°C for 4 hours. To the mixtures was added water, and the resulting mixtures were extracted with ethyl acetate. The organic layers were washed with saturated brine, dried over anhydrous sodium sulfate, and concentrated. The resulting residues were subjected to a column chromatography to give the present compound 2 represented by the following formula 103 mg.

Present compound 2

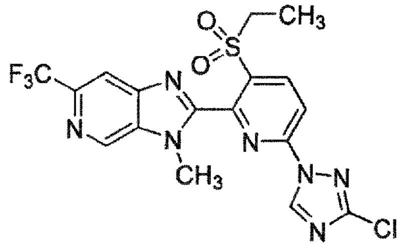

$^1$H-NMR ($CDCl_3$) δ: 9.06 (1H, s), 9.04 (1H, s), 8.73 (1H, d), 8.27 (1H, d), 8.15 (1H, s), 3.93 (3H, s), 3.73 (2H, q), 1.38 (3H, t).
The reaction was carried out using 3-methyl-1H-[1,2,4]-triazole 0.06 g in the place of 3-chloro-1H-[1,2,4]-triazole according to the method described in Preparation Example 2 to give the present compound 3 113 mg. Present compound 3

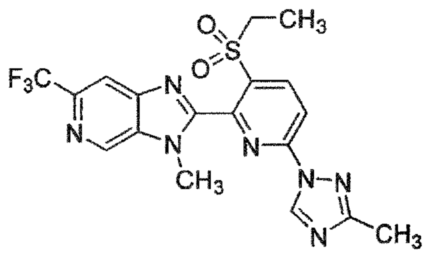

$^1$H-NMR ($CDCl_3$) δ: 9.06-9.01 (2H, m), 8.67 (1H, d), 8.24 (1H, d), 8.14 (1H, s), 3.93 (3H, s), 3.73 (2H, q), 2.55 (3H, s), 1.37 (3H, t).
The reaction was carried out using 3-cyano-1H-[1,2,4]-triazole 0.07 g in the place of 3-chloro-1H-[1,2,4]-triazole according to the method described in Preparation Example 2 to give the present compound 4 32 mg.

Present compound 4

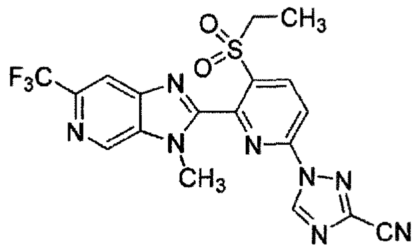

$^1$H-NMR ($CDCl_3$) δ: 9.24 (1H, s), 9.06 (1H, s), 8.81 (1H, d), 8.37 (1H, d), 8.15 (1H, s), 3.94 (3H, s), 3.74 (2H, q), 1.39 (3H, t).
The reaction was carried out using 3-amino-1H-[1,2,4]-triazole 0.06 g in the place of 3-chloro-1H-[1,2,4]-triazole

according to the method described in Preparation Example 2 to give the present compound 5 45 mg.

Present compound 5

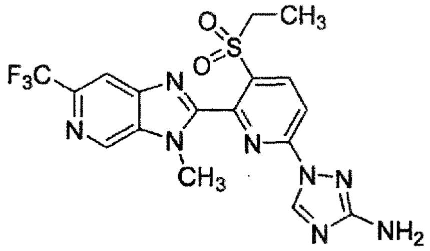

$^1$H-NMR (DMSO-$D_6$) δ: 9.31 (1H, s), 9.05 (1H, s), 8.62 (1H, d), 8.30 (1H, s), 7.95 (1H, d), 6.18 (2H, br s), 3.96 (3H, s), 3.79 (2H, q), 1.20 (3H, t).

The reaction was carried out using 3-nitro-1H-[1,2,4]-triazole 0.08 g in the place of 3-chloro-1H-[1,2,4]-triazole according to the method described in Preparation Example 2 to give the present compound 6 107 mg.

Present compound 6

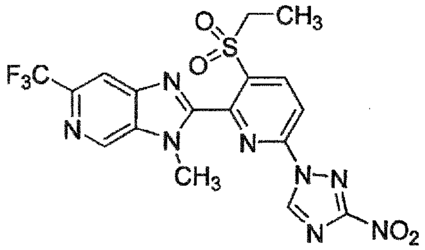

$^1$H-NMR (DMSO-$D_6$) δ: 9.85 (1H, s), 9.35 (1H, s), 8.82 (1H, d), 8.43 (1H, d), 8.34 (1H, s), 4.01 (3H, s), 3.88 (2H, q), 1.23 (3H, t)

Next, the formulation Examples of the present compound are shown below. The "parts" represents "part by weight" unless otherwise specified.

Formulation Example 1

Into a mixture of 10 parts of any one of the present compounds 1 to 6, 35 parts of xylene, and 35 parts of DMF, 14 parts of polyoxyethylene styryl phenyl ether and 6 parts of calcium dodecylbenzene sulfonate are added, followed by mixing them to obtain each formulation.

Formulation Example 2

Four (4) parts of sodium lauryl sulfate, 2 parts of calcium lignin sulfonate, 20 parts of synthetic hydrated silicon oxide fine powder, and 54 parts of diatomaceous earth are mixed, and further 20 parts of any one of the present compounds 1 to 6 is added, followed by mixing them to obtain each wettable powder.

Formulation Example 3

To 2 parts of any one of the present compounds 1 to 6, 1 part of synthetic hydrated silicon oxide fine powder, 2 parts of calcium lignin sulfonate, 30 parts of bentonite and 65 parts of kaolin clay are added, followed by mixing them. To the mixtures is then added an appropriate amount of water, and the mixtures are further mixed, granulated with a granulator, and forced-air drying to obtain each granular formulation.

Formulation Example 4

Into an appropriate amount of acetone, 1 part of any one of the present compounds 1 to 6 is mixed, and then 5 parts of synthetic hydrous silicon oxide fine powder, 0.3 parts of isopropyl acid phosphate, and 93.7 parts of kaolin clay are added, followed by mixing them with stirring thoroughly and removal of acetone from the mixture by evaporation to obtain each of powder formulation.

Formulation Example 5

A mixture of 35 parts of polyoxyethylene alkyl ether sulfate ammonium salt and white carbon (weight ratio of 1:1), 10 parts of any one of the present compounds 1 to 6, and 55 parts of water are mixed, followed by finely grounding them by a wet grinding method to obtain each flowable formulation.

Formulation Example 6

Into a mixture of 5 parts of xylene and 5 parts of trichloroethane, 0.1 parts of any one of the present compounds 1 to 6 is mixed, and the resulting mixture is then mixed with 89.9 parts of kerosene to obtain each oil solution.

Formulation Example 7

Into 0.5 mL of acetone, 10 mg of any one of the present compounds 1 to 6 is mixed, and the solution is added dropwise to 5 g of a solid feed powder for an animal (solid feed powder for rearing and breeding CE-2, manufactured by CLEA Japan, Inc.), followed by mixing the resulting mixtures uniformly. Acetone is then dried by evaporation from the mixtures to obtain each poison bait.

Formulation Example 8

Into an aerosol can, 0.1 part of any one of the present compounds 1 to 6 and 49.9 parts of Neothiozole (Chuo Kasei Co., Ltd.) are placed. After mounting an aerosol valve, 25 parts of dimethyl ether and 25 parts of LPG are filled, followed by shaking the mixtures, and further mounting an actuator to obtain an oily aerosol.

Formulation Example 9

A mixture of 0.6 part of any one of the present compounds 1 to 6, 0.01 part of BHT (2,6-di-tert-butyl-4-methylphenol), 5 parts of xylene, 3.39 parts of deodorized kerosine and 1 part of an emulsifier {Rheodol MO-60 (registered trademark of Kao Corporation)}, and 50 parts of distilled water are filled into an aerosol container, and a valve part is attached. Then, 40 parts of a propellant (LPG) is filled therein through the valve under pressure to obtain an aqueous aerosol.

Formulation Example 10

Zero point one (0.1) parts of any one of the present compounds 1 to 6 are mixed into 2 mL of propylene glycol, and the resulting solution is impregnated into a ceramic plate having a size of 4.0 cm × 4.0 cm and a thickness of 1.2 cm, to obtain thermal fumigants.

Formulation Example 11

Five (5) parts of any one of the present compounds 1 to 6, and 95 parts of ethylene-methyl methacrylate copolymer (the ratio of the methyl methacrylate in the copolymer: 10 weight %), Acryft (registered by trademark) WD 301, manufactured by Sumitomo Chemical Co. Ltd.) are melted and kneaded with a closed type pressure kneader, and the resulting kneaded product is extruded from an extrusion molding machine through a molding die to obtain a rod-shaped molded product having a length of 15 cm and a diameter of 3 mm.

Formulation Example 12

Five (5) parts of any one of the present compounds 1 to 6, and 95 parts of plasticized polyvinyl chloride resin are melted and kneaded with a closed type pressure kneader, and the resulting kneaded product is extruded from an extrusion molding machine through a molding die to obtain a rod-shaped molded product having a length of 15 cm and

a diameter of 3 mm.

Formulation Example 13

One hundred (100) mg of any one of the present compounds 1 to 6, 68.75 mg of lactose, 237.5 mg of corn starch, 43.75 mg of microcrystalline cellulose, 18.75 mg of polyvinylpyrrolidone, 28.75 mg of sodium carbomethyl starch and 2.5 mg of magnesium stearate are mixed, and the resulting mixtures were compressed to an appropriate size to obtain a tablet.

Formulation Example 14

Twenty five (25) mg of any one of the present compounds 1 to 6, 60 mg of lactose, 25 mg of corn starch, 6 mg of carmellose calcium and an appropriate amount of 5% of aqueous hydroxypropyl methylcellulose solution are mixed, and the resulting mixtures are filled into a hard shell gelatin capsule or a hydroxypropyl methylcellulose capsule to obtain capsules.

Formulation Example 15

To 100 mg of any one of the present compounds 1 to 6, 500 mg of fumaric acid, 2,000 mg of sodium chloride, 150 mg of methyl paraben, 50 mg of propyl paraben, 25,000 mg of granulated sugar, 13,000 mg of sorbitol (70% solution), 100 mg of Veegum K (manufactured by Vanderbilt Co.), 35 mg of perfume and 500 mg of coloring agent, a distilled water is added so that a final volume is set to be 100 mL, followed by mixing the mixtures to obtain a suspension for oral administration.

Formulation Example 16

Into a mixture of 5% by weight of an emulsifier, 3% by weight of benzyl alcohol and 30% by weight of propylene glycol, 5% by weight of any one of the present compounds 1 to 6 is mixed, and phosphate buffer is added thereto so that a pH of the solution is set to be 6.0 to 6.5, and water is added as the rest parts thereto to obtain the solution for oral administration.

Formulation Example 17

To a mixture of 57% by weight of fractional distillated palm oil and 3% by weight of polysorbate 85, 5% by weight of aluminum distearate is added, and heated to disperse it. The resulting mixtures are cooled to room temperature, and 25% by weight of saccharin is dispersed in an oil vehicle. Ten (10) % by weight of any one of the present compounds 1 to 6 is divided thereto to obtain a paste for oral administration.

Formulation Example 18

Five (5) % by weight of any one of the present compounds 1 to 6 is mixed with 95% by weight of limestone filler, followed by a wet granulation of the resulting mixture to obtain a granule for oral administration.

Formulation Example 19

Into 80 parts of diethylene glycol monomethyl ether, 5 parts of any one of the present compounds 1 to 6 is mixed, and 15 parts of propylene carbonate is added thereto, and the resulting mixture is mixed to obtain a spot-on solution.

Formulation Example 20

Into 70 parts of diethylene glycol monomethyl ether, 10 parts of any one of the present compounds 1 to 6 is mixed, and 20 parts of 2-octyldodecanol is added thereto to obtain a pour-on solution.

Formulation Example 21

To 0.5 parts of any one of the present compounds 1 to 6, 60 parts of Nikkol (registered by trademark) TEALS-42 (manufactured by Nikko Chemical Co. Ltd.: 42% of aqueous solution of lauryl sulfuric acid triethanol amine) and 20 parts of propylene glycol are added, and the resulting mixture is mixed with stirring thoroughly to obtain a homogeneous

solution, and 19.5 parts of water is then added thereto, and the resulting mixture is further mixed with stirring thoroughly to obtain a homogeneous solution of shampoo formulation.

Formulation Example 22

Zero point fifteen (0.15) % by weight of any one of the present compounds 1 to 6, 95% by weight of animal feed, as well as 4.85% by weight of a mixture of dibasic calcium phosphate, diatomaceous earth, Aerosil, and carbonate (or chalk) are mixed with stirring thoroughly to obtain a premix for animal feed.

Formulation Example 23

Seven point two (7.2) g of any one of the present compounds 1 to 6, and 92.8 g of Hosco (registered trademark) S-55 (manufactured by Maruishi Pharmaceuticals) are melted and mixed at 100°C, and the resulting mixtures was poured into a suppository mold, followed by performing a cooling solidification to obtain a suppository.
Next, Test Examples are used to show an efficacy of the present compound on controlling harmful arthropod pests.

Test Example 1

The test compounds is made to a formulation according to the method described in the Formulation Example 5, and thereto is added water containing 0.03 v/v % of a spreader to prepare a diluted solution containing a prescribed concentration of the test compound.
Cucumber (Cucumis sativus) seedling (on the developmental stage of the second true leaf) is planted in a plastic cup, and approximately 30 heads of cotton aphid (Aphis gossypii) (all stages of life) are released onto the leaves of the cucumber. After 1 day, the diluted solutions were sprayed into the seedling in a ratio of 10 mL/seedling. After 5 days, the number of the surviving insects is examined and the controlling value is calculated by the following equation.

$$\text{Controlling value (\%)} = \{1-(Cb \times Tai)/(Cai \times Tb)\} \times 100$$

wherein the symbols in the equation represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;

Here the "untreated group" represents a group where the similar treatment procedure to that of the treated group except not using the test compound is done.
The results of the test that was conducted according to the method described in Test Example 1 are shown below.
The test was conducted by making the prescribed concentration 500 ppm and using the below-mentioned present compound as a test compound, and as the result of the test, the test compound showed 100 % as the controlling value.

Present compound number: 1

Test Example 2

The test compound is made to a formulation according to the method described in the Formulation Example 5, and thereto is added water containing 0.03 v/v % of a spreader to prepare a diluted solution containing a prescribed concentration of the test compound.
Rice (Oryza sativa) seedling (on the developmental stage of the second true leaf) is planted in a plastic cup, and the diluted solutions are sprayed into the seedling in a ratio of 10 mL/seedling. Thereafter, 20 heads of 3rd instar larvae of brown planthopper (*Nilaparvata lugens*) are released onto the rice leaves. After 6 days, the morality is calculated by the following equation.

Morality (%) = {1- the number of the surviving insects/20} × 100

The results of the test that was conducted according to the method described in Test Example 2 are shown below.
The test was conducted by making the prescribed concentration 500 ppm and using the below-mentioned present compound as a test compound, and as the result of the test, the test compound showed 100 % as the mortality of insects.

Present compound number: 1

Test Example 3

The test compound is made to a formulation according to the method described in the Formulation Example 5, and thereto is added water containing 0.03 v/v % of a spreader to prepare a diluted solution containing a prescribed concentration of the test compound.
The diluted solutions are sprayed into the cabbage (Brassicae oleracea) seedling (on the developmental stage of the second to third true leaf) that is planted in a container in a ratio of 20 mL/seedling. Thereafter, the stem and leaf thereof is cut out and then is installed into the container that is covered with the filter paper. Five (5) heads of cabbage moth (*Plutella xylostella*) at the second instar larval stages are released into the cup. After 5 days, the surviving insects are counted, and the mortality of insects is calculated by the following equation.

Morality (%) = {1- the number of the surviving insects/5} × 100

The results of the test that was conducted according to the method described in Test Example 3 are shown below.
The test was conducted by making the prescribed concentration 500 ppm and using the below-mentioned present compound as a test compound, and as the result of the test, the test compound showed 100 % as the mortality of insects.

Present compound number: 1

Test Example 4

The test compound is made to a formulation according to the method described in the Formulation Example 5, and thereto is added water to prepare a diluted solution containing a prescribed concentration of the test compound.
The bottom of the plastic cup having 5.5 cm diameter is matted with the same size of a filter paper, and 0.7 mL of the diluted solution is added dropwise to the filter paper, and 30 mg sucrose as bait is placed in the cup uniformly. Ten (10) heads of female adult housefly (*Musca domestica*) are released into the plastic cup, and the cup is covered with the lid. After 24 hours, the life and death of housefly is examined and the mortality of insects is determined. The mortality of insects is calculated by the following equation.

Mortality of insects (%) = (Number of dead insects/Number of test insects) × 100

The results of the test that was conducted according to the method described in Test Example 4 are shown below.
The test was conducted by making the prescribed concentration 500 ppm and using the below-mentioned present compound as a test compound, and as the result of the test, the test compound showed 100 % as the mortality of insects.

Present compound number: 1

Test Example 5

The test compound is made to a formulation according to the method described in the Formulation Example 5, and thereto is added water to prepare a diluted solution containing a prescribed concentration of the test compound.

The bottom of the plastic cup having 5.5 cm diameter is matted with the same size of a filter paper, and 0.7 mL of the diluted solution is added dropwise to the filter paper, and 30 mg sucrose as bait is placed in the cup uniformly. Two (2) heads of male German cockroach (*Blattella germanica*) are released into the plastic cup, and the cup is covered with the lid. After 6 days, the life and death of German cockroach is examined and the number of died insects is counted, and the mortality of insects is calculated by the following equation.

Mortality of insects (%) = (Number of dead insects/Number of test insects) × 100

The results of the test that was conducted according to the method described in Test Example 5 are shown below.

The test was conducted by making the prescribed concentration 500 ppm and using the below-mentioned present compound as a test compound, and as the result of the test, the test compound showed 100 % as the mortality of insects.

Present compound number: 1

Test Example 6

The test compound is made to a formulation according to the method described in the Formulation Example 5, and thereto is added water to prepare a diluted solution containing a prescribed concentration of the test compound.

Thirty (30) heads of last instar larvae of common house mosquito (*Culex pipiens pallens*) are released into the diluted solutions and after 1 day, the life and dead of common house mosquito is examined, and the mortality of insects is calculated by the following equation.

Mortality of insects (%) = ((Number of dead insects/Number of test insects) × 100

The results of the test that was conducted according to the method described in Test Example 6 are shown below.

The test was conducted by making the prescribed concentration 3.5 ppm and using the below-mentioned present compound as a test compound, and as the result of the test, test compound showed 91% as the mortality of insects.

Present compound number: 1

Industrial Applicability

The present compound shows an excellent control effect against pests.

## Claims

**1.** A fused heterocyclic compound represented by formula (I) or its N oxide compound:

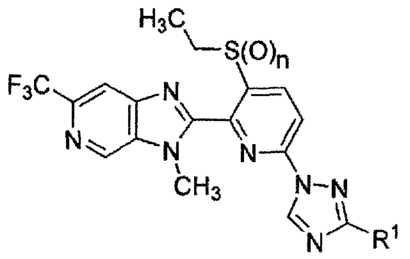

(I)

[wherein,

$R^1$ represents a hydrogen atom, a C1-C3 alkyl group which may have optionally one or more halogen atoms, a halogen atom, a C1-C3 alkoxy group, a C2-C4 alkoxycarbonyl group, $S(O)_mR^2$, $NR^3R^4$, a nitro group, or a cyano group,
$R^2$ represents a C1-C3 alkyl group,
$R^3$ and $R^4$ represent independently of each other a hydrogen atom or a C1-C3 alkyl group, and
m and n are independently of each other 0, 1 or 2.].

**2.** A composition for controlling a pest comprising the compound according to claim 1 and an inert carrier.

**3.** A method for controlling a pest, said method comprising applying an effective amount of the compound according to claim 1 to a pest or a habitat where a pest lives.

## INTERNATIONAL SEARCH REPORT

International application No. PCT/JP2016/082575

A. CLASSIFICATION OF SUBJECT MATTER

*C07D471/04*(2006.01)i, *A01N43/90*(2006.01)i, *A01P7/04*(2006.01)i

According to International Patent Classification (IPC) or to both national classification and IPC

B. FIELDS SEARCHED

Minimum documentation searched (classification system followed by classification symbols)

C07D471/04, A01N43/90, A01P7/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

JSTPlus/JMEDPlus/JST7580(JDreamIII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | CA 2939575 A1 (BAYER CROPSCIENCE AG), 20 August 2015 (20.08.2015), claims; page 24, lines 23 to 24; preparation examples; use examples & WO 2015/121136 A1 & AU 2015217802 A & TW 201620906 A & KR 10-2016-0122809 A | 1-3 |
| P,X | WO 2016/116338 A1 (SYNGENTA PARTICIPATIONS AG), 28 July 2016 (28.07.2016), claims; compounds P16, P21 (Family: none) | 1-3 |
| P,X | WO 2016/124563 A1 (BAYER CROPSCIENCE AG), 11 August 2016 (11.08.2016), claims; examples 5, 15, 18, 32 & UY 36548 A | 1-3 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 06 January 2017 (06.01.17) | 17 January 2017 (17.01.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/082575

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/133603 A1 (Sumitomo Chemical Co., Ltd.), 11 September 2015 (11.09.2015), claims; examples & AU 2015224876 A & CA 2941452 A1 & CN 106103433 A & KR 10-2016-0120748 A & TW 201623299 A | 1-3 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015121136 A
- WO 2010125985 A
- WO 2016116338 A